**Europäisches Patentamt**

**European Patent Office**

**Office européen des brevets**

(19)

(11) Publication number: **0 000 094**

**A1**

(12)

# EUROPEAN PATENT APPLICATION

(21) Application number: 78300023.5

(22) Date of filing: 07.06.78

(51) Int. Cl.²: **B 01 J 8/12**, //C 10 G 35/12

(30) Priority: 09.06.77 US 805062

(43) Date of publication of application:
20.12.78 Bulletin 78/1

(84) Designated Contracting States:
BE CH DE FR GB LU NL

(71) Applicant: UOP INC.,
10 UOP Plaza Algonquin & Mt. Prospect Road,
Des Plaines, Illinois, 60016 (US)

(72) Inventor: Millar, Robert Fredrick,
20, UOP Plaza,
Des Plaines, Illinois (US)

(72) Inventor: Persico, Paul Joseph,
7a Kingery Quarter, Apartement 202,
Hinsdale, Illinoise (US)

(72) Inventor: Jensen, Robert Hugh,
264 Churchill,
Clarendon Hills, Illinois (US)

(74) Representative: Newby, Raymond Laurence,
J.Y. & G.W. Johnson Furnival House 14/18 High Holborn,
GB-London WC1V 6DE (GB)

(54) **Moving bed radial flow solids-fluid contacting apparatus.**

(57) The invention concerns an improved radial flow solids-fluid contacting apparatus (1) in which radial flow of fluid (liquid or gas) coacts with downward flow of the solids in particle form. The invention concerns the design and placement of particle collection scoops (8) close to the bottom of the bed (4) of particles to eliminate stagnant particle zones by improving the conditions under which particles are withdrawn from the bed.

The invention has particular application to a catalytic reactor and an array of arcuate funnel-shaped scoops surrounding the central pipe of such a reactor is preferred. Preferred shapes and angles of inclination to the horizontal for the scoops are disclosed.

EP 0 000 094 A1

- 1 -

## Moving bed radial flow solids-
## fluid contacting apparatus

Radial flow fluid-solids contacting apparatus may be used in a wide variety of industrial processes. Examples are the radial flow reactors used in a variety of hydrocarbon conversion processes. These processes include the isomerization of normal paraffins, the dehydrogenation of normal paraffins and the reforming of naphtha boiling range petroleum fractions. In a radial flow reactor, the various reactants flow along radials extending from the major central axis of the reactor to its periphery. The present invention is concerned with those reactors in which the reactant flow is inwards. That is, the reactants flow inwards from an annular reactant distribution volume to a cylindrical reactant collection volume. This central reactant collection volume is enclosed within an inner catalyst retention screen, which is commonly referred to as the central pipe (or centerpipe) of the reactor. Radial flow may also be used in other contacting apparatus such as adsorbent chambers and treaters. As a corollary to its use as a moving bed reactor, the invention also applies to a moving bed regenerator for the decarbonization, reduction or halogenation of used catalyst or adsorbents.

The term "moving bed" as it is used in this specification is intended to refer to a particulate-containing system in which the particles rest upon one another in a dense bed and the inventory of the bed is gradually replaced through the removal of used particles at the bottom and the addition of fresh or regenerated particles at the top. The present invention is therefore not directed to ebullated or fluidized bed contacting apparatus. Apparatus in accordance with the invention may be used with either vapour or liquid-phase fluids.

It is believed that heretofore, the particles have been withdrawn from annular beds through a number of centrally located openings at the bottom of the vessel. Centrally located is used in this context to indicate the particle or catalyst withdrawal conduits are located approximately midway between the two particle retention screens or within the middle 50% of the distance between the screens. The or each outlet conduit was often covered by a cap or inverted cone such as shown in the specification of United States Patents 3,706,536; 3,785,963 and 3,854,887. The reason for utilizing these caps and the central placement of the withdrawal conduits was to promote a uniform withdrawal of particles from all parts of the bed. That is, it was hoped this prior art structure would effect the withdrawal of bed particles, from zones adjacent to both the screens, at equal rates.

Experience has indicated that these prior art systems do not achieve uniform particle withdrawal. Rather, they may leave a sizable stagnant volume of particles at some location within the apparatus. This stagnant material eventually becomes ineffective in its specific function and exerts a detrimental influence on the performance of the equipment. More specifically, it has been discovered that stagnant particle volumes may occur in the lower portion of the vessel adjacent to the inner retention screen, especially at higher vapour or liquid velocities through the particle bed. Undoubtedly, this is caused at least in part by the inward vapour flow.

A second undesirable effect which has been observed using the prior art particle withdrawal systems is the accumulation of particle fines caused by the normal movement-induced attrition of the particulate material in a zone of stagnant particles. These fines are moved into the stagnant zone by the inward vapour flow but are not removed from it, as would occur if this stagnant zone did not exist. Eventually this transport of fines seals a number of the small vapour passageways in the stagnant zone and causes a higher pressure drop across the bottom of the particle bed than across the top. This in turn distorts the desired equal flow rate through all parts of the annular particle bed. In a reactor, this results in the actual space velocity being higher at the top of the reactor than at the bottom.

This invention seeks to provide a moving bed radial flow solids-fluid contacting apparatus having a minimum of stagnant zones and which lessens the possibility of stagnant zones occurring against the surface of the central pipe of such apparatus during inward vapour flow.

According to the invention a moving bed radial flow solids-fluid contacting apparatus comprises: a vertically oriented outer vessel, a first vertically oriented particle-retaining screen located within the outer vessel to define a fluid distribution volume between the first particle-retaining screen and the outer vessel; a second vertically oriented particle-retaining screen located within the first particle-retaining screen to define therebetween an annular particle retention volume having upper and lower ends, a cylindrical fluid collection volume located within the second particle-retaining screen; a plurality of particle inlet conduits located in an upper section of the outer vessel and communicating with the upper end of the annular particle retention volume; a fluid inlet means communicating with the fluid distribution volume; a fluid outlet means communicating with the cylindrical fluid collection volume; and, a particle withdrawal means located between the first and the second particle retention screens at the lower end of the annular particle retention volume which apparatus is characterised in that the particle withdrawal means comprises: a plurality of tubular particle withdrawal conduits distributed in a pattern encircling the second particle retention screen, each particle withdrawal conduit having an unsealed upper end which communicates with the annular particle retention volume; and, a plurality of particle collection scoops, each scoop having an open upper first end which faces the second particle retention screen and a lower second end which is attached to the upper end of a respective one of each of the particle withdrawal conduits, and with each particle collection scoop having a greater cross-sectional area at the upper first end than at the lower second end and reposing at an angle of from about 5 to 60° with respect to the horizontal.

Preferably the collection scoops substantially completely surround the second particle-retaining screen and define an annular vertically-extending passageway for particles entering the scoops.

The angle of repose can be from 15 to 45° with respect to the horizontal.

The invention will now be further described, by way of example, with reference to the accompanying drawings, in which

Figure 1 is a partially-sectioned side elevation of a reactor in accordance with the invention,

Figure 2 is a view, on an enlarged scale, of part of the reactor of Figure 1,

Figure 3 is a view from above of the reactor part shown in Figure 2,

Figure 4 is a view corresponding to Figure 3 of a modified reactor part, and

Figure 5 is an end view of a part similar to that shown in Figures 2 and 3.

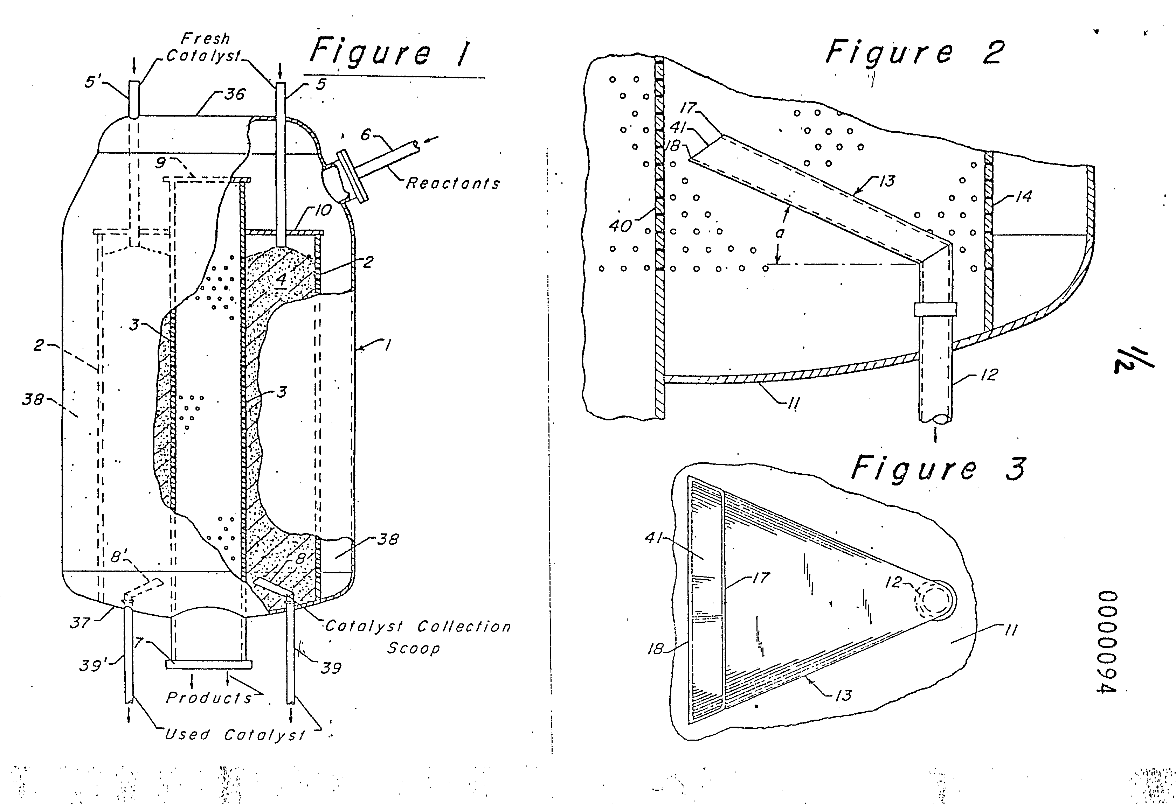

Referring to FIGURE 1, there is shown a moving bed radial flow reactor formed in part by an outer vessel 1 and which conforms to a preferred embodiment of the invention. This vessel is a vertically oriented cylinder enclosed by an upper end section 36 and a lower end section or cap 37. A first or outer catalyst retention screen 2 is also cylindrical in shape, as is an inner or second catalyst retention screen 3. Both of these screens are concentric about the central vertical axis of the reactor. The outer surface of the first screen 2 and the cylindrical inner surface of the outer vessel 1 define an annular fluid or reactant distribution volume 38, into which the reactants flow from an inlet conduit 6. An annular shaped bed 4 of catalyst is retained between the two screens 2 and 3. This catalyst bed is intermittently replenished with fresh catalyst which falls through catalyst inlet conduits 5 and 5'. For simplicity only two catalyst inlet conduits are shown; normally at least about eight are used. The uppermost part of the porous portion of each screen is preferably below the upper inner surface of the vessel, and the top of the screen 2 is sealed off by an imperforate annular plate 10.

The reactants pass inward through the catalyst bed 4 and enter the cylindrical fluid or reactant collection volume within the screen 3. The reactants and reaction products are then removed downwardly from the reactor through the reactant outlet conduit 7. The top of the reactant collection volume is sealed with a circular cover plate 9. Used catalyst is withdrawn from the reactor through a plurality of catalyst collection scoops represented by scoops 8 and 8'. Each scoop has an open upper end which faces the inner catalyst retention screen 3. The catalyst enters through this upper end and is therefor withdrawn in an annular pattern much closer to the inner screen 3 than the outer screen 2. Each scoop has a lower end connected to a catalyst withdrawal conduit such as 39 and 39'. These conduits encircle the inner screen 3 and pass through the lower end section 37. The catalyst collection scoops and the upper portions of the catalyst withdrawal conduits are filled with catalyst during operation of the reactor. This description of the preferred application of the invention is not intended to limit the apparatus to use as a reactor.

In FIGURE 2, a side view of one catalyst collection scoop (here indicated by the numeral 13) is shown in greater detail. The lower end of the scoop 13 is attached to a catalyst withdrawal conduit 12. This vertical conduit extends through an outer wall 11 of the reactor to a series of valves (not shown) or a catalyst collection hopper (not shown) as the case may be. Alternatively, the conduit 12 may directly feed catalyst to another reactor in a "stacked" reactor design. The scoop 13 is positioned such that an opening 41 at the upper end thereof faces the inner catalyst retention screen (here shown as 40) and is adjacent to this screen. The opening 41 therefor faces radially inward of the catalyst bed. The outer catalyst retention screen (here shown as 14) is closer to the lower end of the scoop 13. For simplicity, only a few of the large numbers of perforations in the screens are illustrated. As shown, the scoop 13 is preferably formed by parallel, planar upper and lower imperforate panels, these panels being inclined at an angle "a" to the horizontal as measured from the lower end of the scoop. The sides of the scoop are enclosed by opposing planar side walls.

In FIGURE 3, the view seen when looking downward at a catalyst collection scoop 13 is presented. This scoop is similar to that shown in FIGURE 2. The lower end of the scoop covers the open upper end of the catalyst withdrawal conduit 12. The upper panel of the scoop has an upper edge 17 which is spaced further from the inner catalyst retention screen 40 than the upper edge 18 of the lower panel. The triangular shaped panels result in the scoop having a larger cross-sectional area at its upper end than at its lower end.

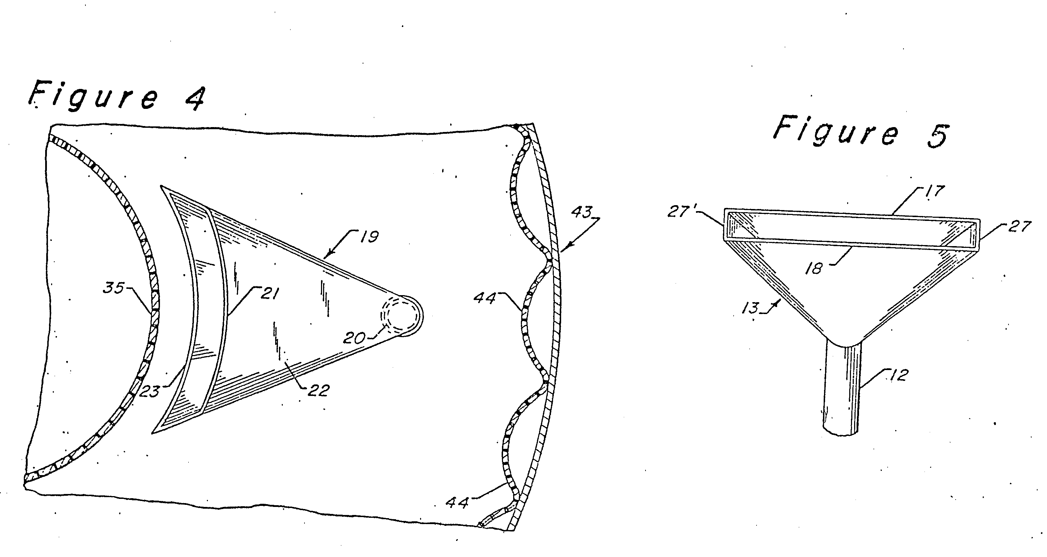

FIGURE 4 illustrates the preferred arcuate shape of the panel edges forming the upper end of a catalyst collection scoop (shown as 19 in this Figure). Again, the lower end of the scoop is positioned to direct catalyst into a withdrawal conduit 20. The upper edges of the panels are once again horizontally staggered to collect catalyst, with an upper edge 21 being positioned further from an inner catalyst retention screen 35 than a lower panel edge 23. In the preferred embodiment, the upper edge of the upper panel 22 and the upper edge of the lower panel are arcuate and have a radius of curvature which is related to that of the inner screen 35 such that the distance measured radially between the inner screen 35 and either upper edge is uniform at all points along each edge (as shown in Figure 4). Figure 4 also shows the use of a scalloped outer catalyst screen 44 adjacent to a cylindrical outer wall 43 of the reactor.

FIGURE 5 represents the view seen when looking towards the upper open end of a catalyst collection scoop 13 which is similar to that shown in Figures 2 and 3. The lower end of the scoop 13 is attached to the catalyst withdrawal conduit 12. The edge 17 of the upper panel is connected to the edge 18 of the lower panel by opposing, vertical, imperforate side walls 27 and 27'. The open upper end of the scoop is therefore circumscribed by the elements 17, 18, 27 and 27'.

The particulate matter used in the apparatus is preferably a hydrocarbon conversion catalyst, but it may be an adsorbent including activated carbon, a zeolite or an alumina as may be used to treat gas streams for the removal of water, sulphur compounds or halogen-containing chemicals. The particulate material may also be a solid acceptor used for the removal of sulphur oxides from a flue gas stream as disclosed in the specifications of United States Patents 3,776,854 and 3,832,445. Preferably, the particulate matter is spherical and has a diameter within the range of 0.15 to 1.25 cm. The words "catalyst" and "particle" are used interchangeably in many instances herein when referring to various elements of the apparatus. The use of either word is not intended to limit the application or function of these elements in a manner which unduly limits the invention. Catalyst used in the apparatus will preferably comprise an inorganic oxide support, such as alumina or silica, with a catalytically effective amount of a metal or metal oxide. The metal may be one or more chosen from nickel, cobalt, iron, platinum, tin, palladium, manganese or magnesium.

The outer vessel is preferably made of a suitable metal such as carbon or stainless steel, but it may be formed from other materials (including fiber reinforced plastics) if the conditions of, for example, temperature and pressure, allow their use.

The two particle or catalyst retention screens are preferably fabricated using wedge-shape wire having a cross-section which decreases in the direction that is away from the particle side of the screen. This results in a self cleaning surface such that as any particles, or pieces thereof, which pass from the bed through the openings between the screen wire fall free of the screen on the side remote from the bed.    Preferably, the wedge-shaped wires are aligned vertically to minimize catalyst attrition as the catalyst moves downward during use against the screen.    The outer particle retention screen (2 in Figure 1) may be either cylindrical, to provide an annular fluid distribution volume 38, or it may be a scalloped screen as shown at 44 in FIGURE 4.

The contact apparatus will be provided with a plurality of particle inlet conduits (5 and 5') which pass through the upper section of the outer vessel and communicate with the top of the bed 4 in the annular particle retention volume formed between the screens. About six to ten inlet conduits are normally adequate to give a proper distribution of the particulate catalyst material across the bed.    The retention screens preferably have an imperforate section at the top to provide a seal which prevents any portion of the fluid stream from passing over the particle bed through possible void spaces.    The screens may be capped as shown in FIGURE 1 or they may be extended upwards to the inner surface of the vessel.    The inlet conduits 5 and 5' will normally be connected to a valve for controlling the rate of particle addition or they may be connected directly to a hopper vessel used to distribute the particles.    Alternatively the inlet conduits may be connected to the outlet conduits of vessels (reactors) located above (as in a stacked design).

In a preferred embodiment the apparatus comprises a plurality of particle collection scoops which are arranged in a circular pattern around, and adjacent to, the bottom of a central screen pipe, such that the tips of the adjacent scoops are close together, or at least within 15 cm of each other, to form an annular catalyst collection zone which is uniformly disposed about the central pipe. The radial width of this zone, which is the same as the distance between the central pipe and the upper edge of one of the scoops, preferably is less than 30 centimeters. Preferably the distance to the upper edge is less than 10 centimeters and is less than 5 centimeters for distance to the lower edge when arcuate scoops are used. An average distance is specified since the straight edge scoops, such as shown in FIGURE 3, will result in a zone of varying radial distance. Preferably, the upper and lower edges of the scoop panels are arcuate and conform closely to the curvature of the central pipe to present a uniform distance between the scoop and the central pipe. Other edge shapes (not shown) may also be employed if desired.

The individual scoops are of hollow form. That is, they are not solid but have a void interior space which conforms to their outer shape. Preferably, the upper and lower panels and side walls which define the scoops and enclose the interior void are imperforate. These elements of the scoops may be relatively porous, however, so as to provide for drainage or vapour circulation. This may be desired in some cases to prevent coking or the entrapment of liquids in the particle withdrawal conduits. The cross-sectional area of the interior of each scoop is larger at the upper end, which faces the central pipe, than at the lower end, which is attached to the particle withdrawal conduit. The scoops are in this regard funnel-like particle collectors. To aid in the collection of the particles, the lower edge of the scoop (the upper horizontal edge of the lower panel) is located closer to the central pipe than the upper edge (see FIGURES 2, 3 and 4.)

The particle collection scoops, or catalyst collection scoops if the apparatus is used as a reactor, are inclined at an angle of from about 5-60° above horizontal. This is indicated as the angle "a" in FIGURE 2. A preferred range for this angle "a" is from 15-45°, with 30° being particularly preferred. It is preferred that the upper and lower panels of the scoop are parallel and therefore have the same angle to the horizontal. This is not a necessity and the scoops may taper in the cross-section contained in a vertical plane also. The scoops may be surrounded by the same particulate material which is moved through the apparatus, or inert spacers such as ceramic spheres may be placed in the bottom of the vessel. Several supports may be placed around and under the scoops to prevent their movement and distortion during the operation of the apparatus.

0000094

- 1 -

<u>CLAIMS</u>

1.    A moving bed radial flow solids-fluid contacting apparatus comprising a vertically oriented outer vessel, a first vertically oriented particle-retaining screen located within the outer vessel to define a fluid distribution volume between the first particle-retaining screen and the outer vessel, a second vertically oriented particle-retaining screen located within the first particle-retaining screen to define therebetween an annular particle retention volume having upper and lower ends, a cylindrical fluid collection volume located within the second particle-retaining screen, a plurality of particle inlet conduits located in an upper section of the outer vessel and communicating with the upper end of the annular particle retention volume, a fluid inlet means communicating with the fluid distribution volume, a fluid outlet means communicating with the cylindrical fluid collection volume, and, a particle withdrawal means located between the first and the second particle retention screens at the lower end of the annular particle retention volume, characterised in that the particle withdrawal means comprises a plurality of tubular particle withdrawal conduits distributed in a pattern encircling the second particle retention screen, each particle withdrawal conduit having an unsealed upper end which communicates with the annular particle retention volume; and, a plurality of particle collection scoops, each scoop having an open upper first end which faces the second particle retention screen and a lower second end which is attached to the upper end of a respective one of each of the particle withdrawal conduits, and with each particle collection scoop having a greater cross-sectional area at the upper first end than at the lower second end and reposing at an angle of from about 5 to 60° with respect to the horizontal.

2. Apparatus of claim 1, characterised in that the collection scoops (8, 13, 19) substantially completely surround the second particle-retaining screen (3, 35,40).

3. Apparatus of claim 1 or claim 2, characterised in that each particle collection scoop (19) has an upper arcuate edge (21) at its upper first end which is substantially uniformly spaced from the second catalyst retention screen (35).

4. Apparatus of claim 3, characterised in that each particle collection scoop (19) has a lower arcuate edge (23) at its upper first end which is also substantially uniformly spaced from the second catalyst retention screen (35) but closer thereto than said upper arcuate edge (21), to provide an annular vertically-extending passageway for particles entering the scoops.

5. Apparatus of any preceding claim characterised in that each particle collection scoop reposes at an angle of from 15 to 45° with respect to the horizontal.

Figure 2

Figure 3

Figure 1

Reactants

Fresh Catalyst

Catalyst Collection Scoop

Products

Used Catalyst

Figure 5

Figure 4

| Category | Citation of document with indication, where appropriate, of relevant passages | Relevant to claim |
|---|---|---|
| DA. | US - A - 3 785 963 (BOYD a. o.) | 1 |
| A | US - A - 2 941 956 (BERGSTROM) | 1 |

**DOCUMENTS CONSIDERED TO BE RELEVANT**

**CLASSIFICATION OF THE APPLICATION (Int. Cl.⁴)**

B 01 J 8/12
// C 10 G 35/12

**TECHNICAL FIELDS SEARCHED (Int.Cl.⁷)**

B 01 J 8/12
B 01 J 8/00
C 10 G 35/12

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 13-09-1978 | MICHIELS |

EPO Form 1503.1 06.78